(19) Europäisches Patentamt
European Patent Office
Office européen des brevets

(11) Numéro de publication : **0 106 749**
**B1**

(12) **FASCICULE DE BREVET EUROPÉEN**

(45) Date de publication du fascicule du brevet :
21.12.88

(21) Numéro de dépôt : 83401905.1

(22) Date de dépôt : 28.09.83

(51) Int. Cl.⁴ : **C 12 Q 1/68**// C12N15/00

(54) Procédé de détection du pouvoir mutagène de substances, susceptibles d'induire la détérioration d'ADN cellulaires, mettant en jeu la production d'une réponse SOS.

(30) Priorité : 28.09.82 FR 8216316

(43) Date de publication de la demande :
25.04.84 Bulletin 84/17

(45) Mention de la délivrance du brevet :
21.12.88 Bulletin 88/51

(84) Etats contractants désignés :
AT BE CH DE FR GB IT LI LU NL SE

(56) Documents cités :
BIOCHIMIE, vol. 64, 1982, pages 797-801 & CNRS
SYMPOSIUM, mai 1982 - Toulouse: "Inducible responses to DNA damages" P. QUILLARDET et al.:
"The SOS chromotest: Direct assay of the expression
of gene sfiA as a measure of genotoxicity of chemicals"
PROC. NATL. ACAD. SCI, vol. 79, octobre 1982, pages
5971-5975, US. P. QUILLARDET et al.: "SOS chromotest, a direct assay of induction of an SOS function in
Escherichia coli K-12 to measure genotoxicity"
NATURE, vol. 290, 30 avril 1981, pages 797-799,
Macmillan Journals Ltd. O. HUISMAN et al.: "An
inducible DNA replication-cell division coupling
mechanism in E. coli"

(73) Titulaire : **INSTITUT PASTEUR**
25-28, rue du Docteur Roux
F-75724 Paris Cédex 15 (FR)

(72) Inventeur : Hofnung, Maurice
15,-rue Bargue
F-75015 Paris (FR)
Inventeur : Quillardet, Philippe
6, rue Berthollet
F-75005 Paris (FR)
Inventeur : Perrin, David
95, bd Saint Michel
F-75005 Paris (FR)
Inventeur : Huisman, Olivier
13 Square Port Royal
F-75013 Paris (FR)
Inventeur : d'Ari, Richard
50 rue des Abesses
F-75018 Paris (FR)

(74) Mandataire : Gutmann, Ernest et al
S.C. Ernest Gutmann - Yves Plasseraud 67, boulevard
Haussmann
F-75008 Paris (FR)

**0 106 749**

## Description

L'invention concerne un procédé de détection du pouvoir mutagène de substances susceptibles d'induire la détérioration D'ADN cellulaire, mettant en jeu la production d'une réponse SOS. Plus particulièrement, elle consiste en un perfectionnement du procédé de détection du caractère éventuellement mutagène de substances, qui fait l'objet de la demande de brevet européen n° 82 400664 (EP-A-0 063 522). Ce procédé consiste à cultiver au sein d'un milieu approprié un micro-organisme contenant dans le chromosome un recombinant d'un gène sfi, notamment sfiA, et d'un gène codant pour une enzyme dosable, avantageusement la β-galactosidase, en présence de la substance étudiée, le caractère mutagène de cette dernière étant évalué par la détection d'un accroissement éventuel de l'enzyme dosable, ou de l'enzyme hybride, constituée au moins en partie par ladite enzyme dosable. Une divulgation orale de ce procédé a été faite par M. Hofnung au cours d'un Symposium du CNRS, qui s'est tenu à Toulouse en mai 1982.

On a cependant constaté que les réponses susceptibles d'être obtenues à l'occasion de la mise en œuvre du procédé de détection décrit dans la demande de brevet européen étaient susceptibles d'être modifiées, au moins pour certaines concentrations de la substance à tester, par exemple lorsque celle-ci s'avère exercer des actions distinctes, indépendantes, sur le micro-organisme étudié, par exemple lorsqu'elle possède la propriété d'induire une inhibition de la synthèse des protéines. Dans un tel cas, l'induction de la β-galactosidase ou de l'enzyme hybride contenant la β-galactosidase peut être suffisam-ment réduite, même lorsque la substance étudiée possède effectivement des propriétés mutagènes, pour rendre aléatoires les conclusions susceptibles d'être tirées des résultats obtenus, au moins en ce qui concerne l'intensité des propriétés mutagènes effective de la substance étudiée.

L'invention a pour but de remédier à ce type de difficultés, plus particulièrement d'obvier aux perturbations éventuelles dues à des propriétés souvent non connues de la substance à l'étude et s'ajoutant à ses propriétés effectivement mutagènes ou génotoxiques, et par conséquent de remédier au masquage éventuel de l'induction de l'enzyme dosable, par lesdites perturbations.

Le procédé selon l'invention pour détecter l'éventuel effet mutagène d'une substance ou composition, qui comprend la réalisation d'une culture d'un micro-organisme, notamment E. coli, hébergeant un recombinant d'un gène sfi et d'un gène codant pour une enzyme dosable, plus particulièrement une fusion sfiA :: opéron lacZ, en présence de la substance à étudier et la mesure de l'activité β-galactosidase, éventuellement induite sous le contrôle du gène sfi, lorsque celui-ci est activé du fait du caractère mutagène de ladite substance ou composition, est caractérisé en ce que l'on mesure également l'activité d'une enzyme distincte, susceptible d'être synthétisée par le micro-organisme en question, cette enzyme étant codée par un gène non impliqué dans les processus d'activation des gènes intervenant dans la production d'une réponse SOS, le caractère éventuellement mutagène de la substance étudiée découlant de la variation, plus particulièrement de l'augmentation du rapport de l'activité de la susdite enzyme dosable à l'activité de l'autre enzyme, notamment eu égard à la valeur observée du même rapport dans une culture du même micro-organisme, mais en l'absence de substance mutagène.

De préférence, l'autre enzyme est choisie parmi celles que le micro-organisme hébergeant le susdit recombinant est capable de produire en quantités significatives. Au besoin, il est rendu constitutif pour ce qui est de la synthèse de l'enzyme choisie. Cette enzyme est avantageusement constituée par la phosphatase alcaline.

Bien entendu, toute autre enzyme que la phosphatase alcaline peut être utilisée comme référence. A titre d'exemple d'enzyme susceptible d'être mise en jeu en lieu et place de la phosphatase alcaline, on citera : la tryptophanase et la glucuronidase.

Cela vaut également en ce qui concerne la susdite enzyme dosable, laquelle peut éventuellement consister en l'enzyme codée par l'un des gènes suivants : UVR A, rec[a] ou umuC.

L'utilisation du rapport susdit comme paramètre à prendre en considération, rend le diagnostic essentiellement indépendant des perturbations auxquelles la synthèse de l'enzyme dosable peut être soumise, en réponse à l'induction du gène sfi, comme conséquence des propriétés distinctes de la substance étudiée et susceptibles d'influencer les synthèses enzymatiques. On a en effet constaté que les perturbations en cause s'exerçaient toujours de façon telle, vis-à-vis tant de l'enzyme dosable induite que de l'enzyme de référence, que le susdit rapport tend de toute façon à s'accroître de façon très notable, lorsque la substance étudiée présente effectivement un caractère mutagène, quels que soient les degrés d'inhibition relatifs des synthèses de l'enzyme dosable, alors induite par l'activation du gène sfi et de l'enzyme de référence.

L'invention concerne également plus particulièrement les souches des micro-organismes hébergeant les susdits recombinants, dans la mesure où ils ont été rendus constitutifs pour ce qui est de la synthèse de l'enzyme de référence. A ce titre, l'invention concerne en particulier les souches de micro-organismes, plus particulièrement de E. coli, hébergeant le recombinant sfiA :: opéron lacZ et autorisant l'expression du gène lacZ, comme suite à l'activation du gène sfiA, ces micro-organismes étant plus particulièrement caractérisés en ce qu'ils sont également constitutifs pour la synthèse de la phosphatase alcaline.

Un micro-organisme hébergeant un recombinant sfi :: opéron lac constitutif pour la synthèse de la phosphatase alcaline peut être obtenu par l'induction d'une mutation dans le gène de régulation phoR ou

2

par transduction du marqueur phoR⁻ au moyen du bactériophage P₁.

D'autres caractéristiques de l'invention apparaîtront encore au cours de la description de modes de mise en œuvre préférés du procédé selon l'invention, en mettant en œuvre une souche également conforme à l'invention, hébergeant un recombinant sfiA :: lac, et rendue constitutive pour la synthèse de la phosphatase alcaline. Référence sera faite au dessin, dans lequel :

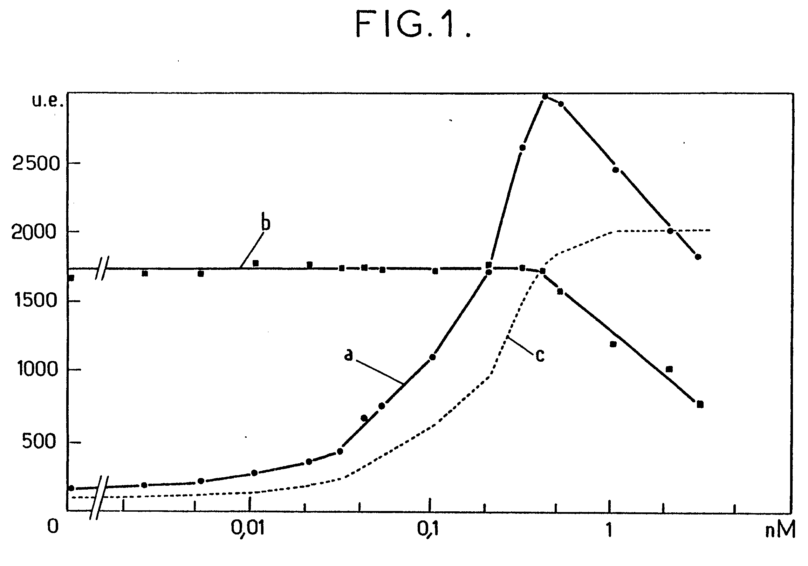

— la fig. 1 contient des courbes représentatives des variations des activités (en unités enzymatiques : u. e.) de la β-galactosidase et de la phosphatase alcaline respectivement, d'une part, et du rapport de l'activité β-galactosidase à l'activité phosphatase alcaline, d'autre part, en fonction de la teneur (en nanomoles : nM du milieu de culture du susdit micro-organisme) en composé carcinogène (ou génotoxique) bien connu, constitué par le 4-nitroquinoline-1-oxyde (4NQO),

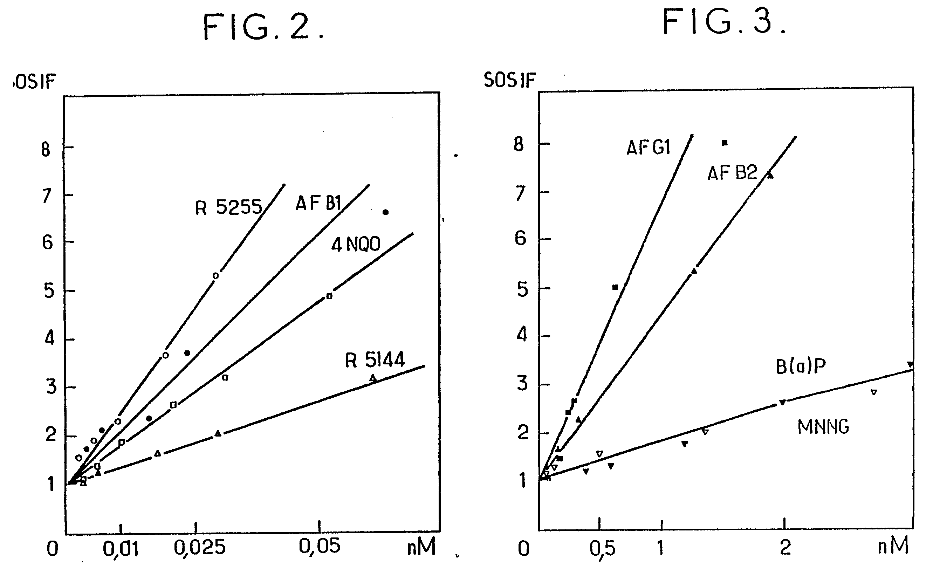

— les fig. 2 et 3 contiennent des courbes représentatives des variations du logarithme du susdit rapport, ci-après dénommées SOSIF (abréviation de « SOS induction factor »), mesurables dans des cultures semblables, en présence de différentes substances génotoxiques, en fonction de leurs concentrations respectives dans les milieux de culture, en nanomoles : par échantillon étudié.

Le micro-organisme mis en œuvre dans les essais dont la description suit est une souche de E. coli transformée, dénommée PQ37, déposée à la Collection Nationale des Cultures de Micro-organismes (C.N.C.M. de l'INSTITUT PASTEUR de Paris, sous le n° I-205 à la date du 24 septembre 1982. Les caractéristiques génétiques de ce micro-organisme sont les suivantes : F⁻ thr leu his-4 pyrD thi qalE galK ou T lacΔUI69 srl300 :: Tn10 rpoB rpsL uvrA rfa trp :: Muc⁺ sfiA :: Mud (Ap, lac) c-Ts. Il est constitutif pour la synthèse de l'alcaline phosphatase (Pho°).

Cette souche a été obtenue par les recombinaisons génétiques appropriées pour lui conférer le génotype sus-indiqué, à partir de la souche contenant le recombinant sfiA :: lacZ déposé à la C.N.C.M. sous le n° 1-152 le 13 avril 1981 (voir EP-A-0 063 522).

Le procédé selon l'invention peut notamment être mis en oeuvre dans les conditions suivantes :

Une culture du susdit micro-organisme préalablement développé jusqu'à la phase exponentielle dans le milieu de culture LB (Bacto® tryptone 10 g, Bacto® yeast extract 5 g, NaCl 10 g, eau Q.S.P. 1 l), contenant également de l'ampicilline, est diluée dix fois soit dans du milieu frais, soit dans le mélange d'activation décrit par AMES B. N. et Coll. (1975), « Mutation Research » 31, 347-364. Des aliquotes de 0,6 ml sont réparties dans des tubes à essais en verre contenant chacun une dose déterminée de la substance à tester. Après deux heures d'incubation à 37°C sous agitation, les activités β-galactosidase et phosphatase alcaline sont dosées.

On peut en particulier effectuer le dosage de la phosphatase alcaline dans les conditions suivantes :

2,7 ml de tampon T (solution de 121 g de Tris (hydroxyméthyl)-aminométhane par litre, dont le pH a été ajusté à 8,8 avec HCl) sont ajoutés à 0,3 ml de la culture des cellules. Les membranes cellulaires sont rompues par addition d'une solution de dodécyl-sulfate de sodium à 0,1 % et de 0,15 ml de chloroforme et par agitation vigoureux dans un agitateur du type VORTEX. Les tubes sont ensuite amenés à la température stabilisée de 28°C. La réaction débute par addition de 0,6 ml d'une solution de paranitrophénylphosphate (PNPP)(4 mg/ml dans le tampon T). Elle est ensuite arrêtée par addition de 1 ml de HCl 2N. 5 minutes plus tard, 1 ml de Tris 2 N est ajouté pour restituer la couleur, laquelle est mesurée au spectrophotomètre à 420 nm. Les unités enzymes sont calculées comme pour la β-galactosidase, selon la méthode décrite par MILLER J. (1972) « Cold Spring Harbour Laboratory », New York.

Le dosage de l'activité β-galactosidase est effectué selon la méthode également décrite par MILLER. Le protocole opératoire est le même que pour la phosphatase alcaline, sauf que le tampon T est remplacé par le tampon Z dont la composition est indiquée ci-après et que l'on utilise à la place du PNPP le substrat constitué par (ONPG) et enfin que la réaction est interrompue avec du carbonate de sodium.

La composition du tampon Z est la suivante par litre) :

| | |
|---|---|
| $Na_2 HPO_4, 7H_2O$ | 16,1 g |
| $NaH_2PO_4, H_2O$ | 5,5 g |
| KCl | 0,75 g |
| $MgSO_4, 7H_2O$ | 0,25 g |
| β-mercaptoéthanol, pH ajusté à 7,0 | 2,7 ml |
| pH ajusté à 7,0. | |

Les courbes de la fig. 1 sont représentatives des variations, en fonction des concentrations en substance génotoxique (4NQO) :

— de l'activité β-galactosidase (courbe a) ;
— de l'activité de la phosphatase alcaline (courbe b) ;
— du rapport SOSIF (courbe c).

Les différentes mesures ont été effectuées 2 heures après le début de l'expérience. En effet, l'expérience montre que la courbe représentative du facteur SOSIF, pour toute concentration en substance génotoxique, atteint un plateau au bout de 70 à 100 minutes, et reste stable en général pendant plus de 2 heures. Les courbes montent en outre, au-delà d'une certaine concentration en substance génotoxique qu'il s'ensuit un effet inhibiteur de la synthèse des protéines se manifestant par une réduction des activités β-galactosidase et phosphatase alcaline. Cependant, le rapport SOSIF, utilisé pour l'appréciation de l'effet mutagène ou génotoxique de la substance étudiée, n'est pratiquement pas affecté. En effet, il reste sensiblement constant à des concentrations de la substance génotoxique qui sont de nature à inhiber la synthèse des protéines.

Les courbes des fig. 2 et 3 sont représentatives des variations du rapport SOSIF en fonction des concentrations utilisées en divers agents génotoxiques.

Les substances génotoxiques utilisées ont été les suivantes :

R 5255 : 2-nitro-5-méthoxybenzofuranne

AF B1 : aflatoxine

4NQO : 4-nitroquinoline-1-oxyde

R 5144 : 2-nitro-benzofuranne

AF G1 : aflatoxine G

AF B2 : aflatoxine B

B (a) P : benzo-a-pyrène

MNNG : N-méthyl-N'-nitro-N-nitrosoguanidine

Il est particulièrement remarquable que le facteur SOSIF soit en fonction sensiblement linéaire de la concentration de la substance génotoxique utilisée, en particulier pour des doses contenues dans un intervalle de doses relativement faibles. La sensibilité du test est particulièrement remarquable, si l'on observe que les variations des valeurs mesurées s'étendent dans un intervalle de 1 à 60 millions, lorsque l'on passe de la limite inférieure à la limite supérieure de l'intervalle des concentrations exprimées en abscisse.

L'invention fournit par conséquent un test d'une sensibilité tout à fait remarquable, apte à donner des indications quantitatives précises, eu égard à l'intensité de l'effet mutagène des substances étudiées.

Comme il va de soi et comme il résulte d'ailleurs déjà de ce qui précède, l'invention ne se limite nullement à ceux de ses modes d'application et de réalisation qui ont été plus spécialement envisagés ; elle en embrasse au contraire toutes les variantes.

## Revendications

1. Procédé pour détection de l'éventuel effet mutagène d'une substance ou composition, qui comprend la réalisation d'une culture d'un micro-organisme, notamment E. coli, hébergeant un recombinant d'un gène sfi et d'un gène codant pour une enzyme dosable, plus particulièrement une fusion sfiA : opéron lacZ, en présence de la substance à étudier et la mesure de l'activité β-galactosidase, éventuellement induite sous le contrôle du gène sfi, lorsque celui-ci est activé du fait du caractère mutagène de ladite substance ou composition, caractérisé en ce que l'on mesure également l'activité d'une enzyme distincte, susceptibles d'être synthétisée par le micro-organisme en question, cette enzyme étant codée par un gène non impliqué dans les processus d'activation des gènes intervenant dans la production d'une réponse SOS, le caractère éventuellement mutagène de la substance étudiée découlant de la variation observée, plus particulièrement de l'augmentation du rapport de l'activité de la susdite enzyme dosable à l'activité de l'autre enzyme, notamment eu égard à l'évolution du même rapport dans une culture du même micro-organisme, mais en l'absence de substance mutagène.

2. Procédé selon la revendication 1, caractérisé en ce que l'enzyme dosable est constituée par la β-galactosidase.

3. Procédé selon les revendications 1 ou 2, caractérisé en ce que l'enzyme distincte est constituée par la phosphatase alcaline.

4. Procédé selon l'une quelconque des revendications 1 à 3, caractérisé en ce que le micro-organisme a été rendu constitutif pour la synthèse de l'enzyme distincte.

5. Micro-organisme, particulièrement E. coli, hébergeant le recombinant sfiA :: opéron lacZ et autorisant l'expression du gène lacZ, comme suite à l'activation du gène sfiA, ce micro-organisme étant plus particulièrement caractérisé en ce qu'il est également constitutif pour la synthèse de la phosphatase alcaline.

## Claims

1. Process for the detection of a possible mutagenic effect of a substance or a composition which comprises making a culture of a microorganism, particularly E. coli, which harbors a recombinant of sfi gene and of a gene coding for a dosable enzyme, more particularly a fusion sfiA : operon lacZ, in the presence of the substance under study and the measurement of the β-galactosidase activity possibly induced under the control of the sfi gene, when the latter is activated as a result of the mutagenic character of said substance or composition, characterized in that one measures also the activity of a distinct enzyme liable of being synthesized by the microorganism under consideration, this enzyme being coded by a gene which is not involved in the activation process of the genes which take part into the production of SOS response, the possibly mutagenic character of the substance under study resulting from the variation observed more particularly of the increase of the ratio of the activity of said dosable enzyme to the activity of the other enzyme, particularly with respect to the variation of the same ratio in a culture of the same microorganism, however in the absence of the mutagenic substance.

2. Process according to claim 1, characterized in that the dosable enzyme is constituted by the β-galactosidase.

3. Process according to claim 1 or 2, characterized in that the distinct enzyme is constituted by alkaline phosphatase.

4. Process according to anyone of claims 1 to 3, characterized in that the microorganism has been made constitutive for the synthesis of the distinct enzyme.

5. Microorganism, particularly E. coli, that harbors the sfiA :operon lacZ recombinant and which authorizes the expression of lacZ gene, as a follow up to the activation of the sfiA gene, said microorganism being more particularly characterized in that it is also : constitutive for the synthesis for the alkaline phosphatase.

**Patentansprüche**

1. Verfahren zum Nachweis der möglichen mutagenen Wirkung eines Stoffes oder einer Zusammensetzung, bei dem man einen Mikroorganismus, insbesondere E. coli, der eine Rekombination des Gens sfi mit einem für ein dosierbares Enzym codierenden Gen, insbesondere die Fusion sfiA :: lacZ-Operon enthält, in Gegenwart des zu untersuchenden Stoffes züchtet und die gegebenenfalls unter der Kontrolle des sfi-Gens induzierte β-Galaktosidase-Aktivität mißt, wenn diese durch die mutagenen Eigenschaften des Stoffes oder der Zusammensetzung erhöht ist, dadurch gekennzeichnet, daß man ebenfalls die Aktivität eines unterscheidbaren Enzyms mißt, das vom Mikroorganismus synthetisiert werden kann und von einem nicht in die Aktivierungsprozesse der die SOS-Antwort bildenden Gene einbezogenen Gen codiert wird, wobei sich die möglichen mutagenen Eigenschaften des untersuchten Stoffes aus der beobachteten Veränderung ergeben, insbesondere aus dem Anstieg des Verhältnisses der Aktivität des dosierbaren Enzyms zur Aktivität des anderen Enzyms, insbesondere im Vergleich zur Entwicklung dieses Verhältnisses in einer Kultur des gleichen Mikroorganismus, die keinen mutagenen Stoff enthält.

2. Verfahren nach Anspruch 1, dadurch gekennzeichnet, daß das dosierbare Enzym die β-Galaktosidase ist.

3. Verfahren nach Anspruch 1 oder 2, dadurch gekennzeichnet, daß das unterscheidbare Enzym die alkalische Phosphatase ist.

4. Verfahren nach einem der Ansprüche 1 bis 3, dadurch gekennzeichnet, daß der Mikroorganismus konstitutiv für die Synthese des unterscheidbaren Enzyms gemacht wurde.

5. Mikroorganismus, insbesondere E. coli, der die Rekombination sfi :: lacZ-Operon enthält und das lacZ-Gen infolge der Aktivierung des sfiA-Gens exprimieren kann, wobei der Mikroorganismus insbesondere dadurch gekennzeichnet ist, daß er gleichzeitig auch die alkalische Phosphatase konstitutiv synthetisiert.

FIG.1.

FIG. 2.

FIG. 3.